# EUROPEAN PATENT SPECIFICATION

(11) **EP 4 149 428 B1**
(45) Date of publication and mention of the grant of the patent: **28.08.2024**
(21) Application number: 21739173.9
(22) Date of filing: 12.05.2021
(51) Int. Cl.: A61K 9/00, A61K 47/12, A61P 7/08

(54) **COMBINED HYPEROSMOLAR SOLUTION FOR INFUSION**
KOMBINIERTE HYPEROSMOLARE LÖSUNG ZUR INFUSION
SOLUTION HYPEROSMOLAIRE COMBINÉE POUR PERFUSION

(30) Priority: 12.05.2020 UA 202002870; 22.05.2020 UA 202003063 U
(43) Date of publication of application: 22.03.2023
(73) Proprietor: M.T.K. Medical Center Limited Liability Company, Kyiv 03680 (UA)
(72) Inventor: GUMENIUK, Mykola Ivanovych, Kyiv, 03110 (UA); DERKACH, Nataliia Mykolaiivna, Kyiv, 03110 (UA); KONDRATSKYI, Bohdan Oleksiiovych, Lviv, 79013 (UA); DERKACH, Dmytro Ivanovych, Kyiv, 03110 (UA)
(74) Representative: Batakliev, Dimitar
(86) International application number: PCT/IB2021/054055
(87) International publication number: WO 2021/229467

(56) References cited:
- WO-A1-97/41848
- UA-C2- 93 776
- ADAMS H A ET AL: "Infusionstherapie im Rettungsdienst", INTENSIVMEDIZIN + NOTFALLMEDIZIN ; GERMAN INTERDISCIPLINARY JOURNAL OF INTENSIVE CARE MEDICINE, STEINKOPFF-VERLAG, DA, vol. 47, no. 5, 27 May 2010 (2010-05-27), pages 370 - 380, XP019846539, ISSN: 1435-1420

## Description

### TECHNICAL FIELD

The invention relates to the field of medicine, specifically to hyperosmolar parenteral medicinal products in a dosage form of solutions for infusion.

### BACKGROUND ART

Solutions for infusion - medicinal products that are administered into the human body by intravenous infusion and using infusion systems. The main indications for prescription of infusion therapy are:
- insufficient blood volume;
- deterioration of blood rheological properties;
- deterioration of water-salt and acid-base blood balance;
- severe dehydration;
- intoxication, etc.

According to the therapeutic effect, solutions for infusion are conventionally divided into several groups.

The first group includes the solutions for infusion with hemodynamic effect. Hemodynamic solutions for infusion (other terms found in the literature - anti-shock and volemic) act as plasma substitutes, and restore the volume, hemodynamics and blood circulation in blood loss and shock. Water-soluble polymers such as gelatin and hydroxyethyl starch (HES) are used as active pharmaceutical ingredients (API) in the hemodynamic solutions for infusion, wherein the therapeutic effect of the solutions for infusion (duration of its circulation in the blood stream and hemodynamic effect) directly depends on the characteristics of the polymer. In the current medical practice, medium and low molecular weight polymers, which are characterized by less pronounced side effects, are used most often.

However, the hemodynamic solutions for infusion have disadvantages including a narrow therapeutic effect. Therefore, they are mainly used only to correct the volemic status of the patients.

In addition, the disadvantage of the hemodynamic solutions for infusion containing HES is a pronounced negative effect on the renal function, among other side effects. For example, very often, use of STABIZOL^{®} solution for infusion (see web page at https://compendium.com.ua/dec/271727/) containing HES with an average molecular weight of 45,0000 Daltons and a degree of molar substitution of 0,7, and sodium chloride, accompanies cases of pain in the kidneys, which leads to discontinuation of the therapy and impossibility of further use of the medicinal product.

Also, disadvantages of the hemodynamic solutions for infusion containing gelatin include allergic reactions of varying severity, even up to the development of anaphylactic shock. A well-known representative of this class of the medicinal products is GELOFUSINE solution for infusion (see web page at http://likicontrol.com.ua/%D1%96%D0%BD%D1%81%D1%82%D1%80%D1%83%D0% BA%D1%86%D1%96%D1%8F/?[26777]) containing as API medium molecular weight succinylated gelatin (average molecular weight 30,000 Da) and sodium chloride. Highlights of prescribing information for the GELOFUZINE medicinal product states that severe anaphylactoid reactions (III or IV degree) are very rare (frequency <1 :10,000). However, the risk of such severe side effects cannot be eliminated, therefore, patients receiving GELOFUSINE need constant medical supervision on the subject of occurrence allergic reactions.

The second group includes solutions for infusion for regulation water-salt and acid-base blood balance. They are used to replenish blood loss and stabilize the circulating plasma volume, improve the blood rheological properties, normalize the blood ionic composition and pH, as well as general rehydration and detoxification. In fact, the solutions for infusion of this group are saline solutions that do not contain polymeric components.

The following representatives of the second group of solutions for infusion are known. ACESOL solution for infusion (see web page at http://likicontrol.com.ua/%D1%96%D0%BD%D1%81%D1%82%D1%80%D1%83%D0% BA%D1%86%D1%96%D1%8F/?[14778]) contains 200 mg of sodium acetate trihydrate, 500 mg of sodium chloride, and 100 mg of potassium chloride per 100 ml of the solution. The disadvantage of this medicinal product is the high content of potassium salts, which can lead to the development of hyperkalemia. During massive infusions of ACESOLUM medicinal product, the patient may develop metabolic disorders, that leads to the need of additional sodium medicinal product administration to correct the electrolyte balance of the patient blood.

Another known second group solution for infusion is LACTASOL (see web page at https://www.piluli.kharkov.ua/drugs/drug/1544/). LACTASOL contains sodium chloride 0,62 % wt., potassium chloride 0,03 % wt., calcium chloride 0,016 % wt., magnesium chloride 0,01 % wt., sodium lactate 0,336 % wt., sodium bicarbonate 0,03 % wt. The ionic composition of the medicinal product is following: Na⁺ - 140,0 mmol/L, K⁺ - 4,0 mmol/L, Ca⁺⁺ - 1,5 mmol/L, Mg++ - 1,0 mmol/L, Cl⁻ - 116 mmol/L, HCO₃⁻ - 4,0 mmol/L, CH₃CH(OH)SOO⁻ - 30,0 mmol/L, osmolarity - 294 mOsm/L. The disadvantage of the medicinal product is in its instability after sterilization due to the presence of sodium bicarbonate. Additionally, the amount of sodium lactate in the medicinal product is negligible. Therefore, when using LACTASOL medicinal product to correct blood acid-base balance, it is necessary to transfuse significant amounts of the medicinal product, which leads to overload of the body blood stream.

Also is known STEROFUNDIN ISO solution for infusion (see web page at http://mozdocs.kiev.ua/likiview.php?id=24436) that contains a combination of the alkalizing component, such as sodium acetate, and L-malic acid, and contains sodium ion - 145,0 mmol/L, potassium ion - 4,0 mmol/L, calcium ion - 2,5 mmol/L, magnesium ion - 1,0 mmol/L, chloride ion - 127,0 mmol/L, acetate ion - 24,0 mmol/L and malate ion - 5,0 mmol/L.

STEROFUNDIN ISO solution for infusion, as well as the above-mentioned solutions for infusion of the second group are saline isoosmolar solutions, and, therefore, have very limited pharmacological action.

One more known saline solution for infusion - HARTMAN'S SOLUTION (see web page at http://likicontrol.com.ua/%D1%96%D0%BD%D1%81%D1%82%D1 %80%D1%83%D0% BA%D1%86%D1%96%D1%8F/?[24214]) containing sodium chloride, potassium chloride, sodium lactate, calcium chloride dihydrate, magnesium chloride hexahydrate. 100 ml of the medicinal product contains sodium chloride - 0,6 g; potassium chloride - 0,04 g; sodium lactate - 0,303 g; calcium chloride dihydrate - 0,2013 g; magnesium chloride hexahydrate - 0,02 g. The disadvantage of HARTMAN'S SOLUTION medicinal product is in its low osmolarity. The medicinal product is a hypotonic solution, which significantly limits its use, because the use of this medicinal product in large doses can lead to intracellular edema of the internal organs and, mainly, to cerebral edema and increased intracranial pressure of the patient. In case of severe oxygen deficiency, HARTMAN'S SOLUTION can worsen the developing lactic acidosis.

Another known saline solution for infusion is DARROW'S SOLUTION (see web page at http://mozdocs.kiev.ua/likiview.php?id=1526). 100 ml of the medicinal product contains sodium chloride - 0,4 g; potassium chloride - 0,267 g; sodium lactate - 0,594 g. DARROW'S SOLUTION has a limited range of applications. Also, the disadvantage of the medicinal product is in its high potassium content, which leads to a high risk of hyperkalemia and the need for strict control of potassium levels in the patient's serum. DARROW'S SOLUTION is an unbalanced solution because it contains only sodium and potassium ions as cations, therefore, the composition of the medicinal product differs significantly from the composition of the blood plasma, which can lead to acid-base and water-electrolyte disbalances when using this unbalanced solution for infusion.

The third group of the solutions for infusion includes solutions for infusion with combined (complex) action, that can be used as hemodynamic solutions, detoxifying solutions, and solutions for regulating water-salt and acid-base blood balance. Currently, combined solutions for infusion are the most promising, as they can be used to treat a wide range of diseases and pathological conditions.

There is a known combined solution for infusion RHEOSORBILACT^{®} (see web page at https://compendium.com.ua/dec/267128/)that contains polyol, such as sorbitol, in an amount of 6 % wt., sodium lactate - 1,9 % wt., sodium chloride - 0,6 % wt., potassium chloride - 0,03 % wt., calcium chloride - 0,01 % wt., magnesium chloride - 0,02 % wt. The ionic composition of the medicinal product is following: Na⁺ - 278,2 mmol/L, K⁺ - 4,0 mmol/L, Ca⁺⁺ - 0,9 mmol/L, Mg++ - 2,1 mmol/L, Cl⁻ - 112,7 mmol/L, CH₃CH(OH)SOO⁻ - 175,5 mmol/L. The osmolarity of the medicinal product is 891 mOsm/L. RHEOSORBILACT^{®} has plasma-substituting and detoxifying properties, and is prescribed for the use in the following cases:
- the treatment of shock microcirculation disorders;
- various disorders of peripheral blood circulation;
- correction of the water-salt balance;
- the combined treatment of intestinal obstruction;
- infectious diseases that are accompanied by severe intoxication, repeated vomiting and diarrhea;
- in surgical practice for the treatment of systemic inflammatory response syndrome (sepsis);
- the treatment of the biliary tract diseases, such as gallbladder dyskinesia, postcholecystectomy syndrome;
- as a part of the combined conservative therapy of acute pancreatitis;
- in neurological practice, in particular, for the treatment of cerebral ischemia and diabetic neuropathy.

Microcirculation - a generic term that includes blood flow and lymph flow in microvessels, as well as plurality of metabolic processes that occur through the walls of microvessels, and extravascular transport of fluids and substances. The main function of the microcirculation is metabolic. Continuous blood flow ensures the excretion of waste products and the supply of nutrients to body tissues. Another important function of the microcirculation is maintaining pressure by ensuring the balance of fluid entering or leaving tissues and vessels.

The microcirculatory blood stream consists of vessels whose diameter does not exceed 100 µm (arterioles, venules, capillaries, etc.).

Microcirculation disorders are divided into the following types:
- extravascular disorders, such as changes in perivascular transport of interstitial fluid, degranulation of tissue basophils, and release of biologically active substances and enzymes into the surrounding vascular tissue;
- intravascular disorders, such as changes in blood viscosity and coagulation, erythrocyte sludge, and blood velocity;
- disorders of microvessels walls, such as changes in vascular permeability and diapedesis of blood cells.

The microcirculation disorder is a result of many diseases, and a dangerous symptom, as it leads to metabolic disorders and abnormal functioning of the tissues and organs. The main reasons for the development of the microcirculation disorders are inflammatory processes, hyperproteinemia, anhydremia, hypoxia, acidosis, intoxication, hypovolemia, hypervolemia, shock. Some of these diseases require immediate treatment because they are life-threatening (e.g., shock).

Shock - is an acute pathological process that occurs because of insufficient blood supply to the organs and tissues and can lead to the death. Circulatory disorders in the body and shock can develop for the following reasons:
- reduction of the total blood volume in the body (e.g., as a result of blood loss);
- sudden dilation of the blood vessels and an increase in their volume (due to allergic reactions, pain);
- heart disorders (myocardial infarction);
- inflammatory processes (sepsis).

In the United States, about 1,2 million people check into the emergency unit each year in a state of shock, wherein the risk of death is very high, ranging from 20 to 50%.

Peripheral circulation is a movement of blood within one organ. Among the main peripheral circulation disorders are following:
1) ischemia that is a decrease in blood flow in an organ or tissue due to a decrease in blood supply by the artery;
2) arterial hyperemia that is an increase in blood flow in an organ or tissue due to the expansion of the artery lumen and the increase in blood flow from it;
3) venous hyperemia that is an increase in the filling of an organ or tissue with blood due to impaired blood flow through the veins;
4) thrombosis that is a deterioration of the blood properties resulting in a clot in the vessel lumen;
5) embolism that is a closure of the vessel lumen due to the movement of foreign substrates (emboli) in the blood stream;
6) bleeding that is an outflow of blood from the blood vessels due to impairment of the integrity or increased permeability of the vessel wall.

A severe consequence of peripheral circulatory disorders is an infarction, meaning necrosis of an organ or its part, which develops due to circulatory disorders in it.

Intoxication is a process of organ damage due to the harmful effects of poisons and toxins. Intoxication can occur for various reasons, such as an entry of the harmful substances into the body from the outside, or their formation inside the body. Intoxication can also be caused by diseases, such as infectious diseases, when the poisoning of the body is caused by the products of pathogens vital activity. One of the mechanisms to combat the effects of intoxication is detoxification by administering solutions for infusion into the blood stream (accelerating the excretion of the harmful substances from the body through the water load of the body).

Sepsis is a dysfunction of the organs due to the development of a systemic inflammatory response syndrome. Sepsis occurs in the body due to infectious diseases caused by various pathogens. Effective methods of treating septic conditions include a combined therapy with using infusion medicinal products for detoxification, hemodynamic correction, parenteral nutrition, blood transfusions and others.

Thus, the complex mechanism of action of the known solution for infusion RHEOSORBILACT^{®} contributes to its multi-purpose use in the clinical practice. However, RHEOSORBILACT^{®} medicinal product is not all-purposed. Thus, the general somatic contraindications for the use of RHEOSORBILACT^{®} medicinal product include alkalosis of any genesis (respiratory, metabolic, etc.), disseminated intravascular coagulation (DIC) syndrome, cerebral hemorrhage, gastrointestinal bleeding with unstable hemostasis, acute thrombosis (due to a risk of thromboembolic complications). It is not recommended to use RHEOSORBILACT^{®} for first aid in injuries until the final cessation of bleeding (due to the ability to reduce blood viscosity the medicinal product may interfere with blood clotting). Contraindications also include severe forms of heart failure and acute renal failure (in the absence of hemodialysis).

The disadvantages of the known medicinal product also include:
- one of the components of RHEOSORBILACT^{®} medicinal product, such as sodium lactate, is contained in large enough quantities, which can cause overload of the body with lactate and the load on the liver during the metabolism of large amounts of lactate;
- insufficient speed of onset of action;
- the possibility of developing side effects when using the known medicinal product for the treatment of patients with fructose intolerance;
- insufficient effectiveness of its action in the treatment of the microcirculation and peripheral blood circulation disorders.

The objective technical problem to be solved is to develop a medicinal product in a dosage form of solution for infusion having a combined action, being effective and safe for the treatment of the disorders of microcirculation and peripheral circulation, for restoration of water-salt balance, rheological properties of the blood, and capillary tonus in different population groups, including patients suffering from diabetes and fructose intolerance; being characterized by a faster onset of an alkalizing action, reduced risk of overdose of the patient's body with lactate ion; and being used to implement a restrictive infusion strategy.

### SUMMARY OF INVENTION

The objective technical problem is solved by introducing into the composition of the solution for infusion two additional reserve alkalinity carriers, that allows to reduce the sodium lactate content in the final composition of the solution for infusion while maintaining the required osmolarity of the solution and achieving additional benefits that expand the spectrum of its action. As additional reserve alkalinity carriers, it is proposed to use a mixture of sodium acetate and L-malate in an amount calculated in order to maintain the optimal value of the total osmolarity of the hyperosmolar solution for infusion. At the same time, 4 times higher amounts of lactate in the disclosed solution for infusion were reached, compared to the existing analogues, and the total amount of lactate is lower compared to the closest prototype (RHEOSORBILACT^{®}), that enables achieving the object of using the solution for infusion in situations where a patient is at risk of lactate ion overdose.

The objective technical problem is also solved by the balanced content of three reserve alkalinity carriers in the disclosed solution for infusion. Since lactate ions are metabolized in the liver and acetate and malate ions - in muscle, reducing the total amount of lactate ions in the solution for infusion reduces the load on the liver when using the disclosed solution for infusion. In addition, the use of the components of the solution for infusion, containing acetate and malate anions, as carriers of the reserve alkalinity, allows minimizing the oxygen consumption in the tissues that is required for the formation of bicarbonate, which is very important for patients in shock who develop respiratory insufficiency.

Also, additional objective technical problems are solved, such as effective correction of metabolic acidosis and enhancement of antihypoxant and detoxifying effects of the solution for infusion due to the successful use and scientifically selected and calculated amounts of malate and acetate ions introduced into the solution for infusion; faster onset of action and manifestation of the alkalizing effect due to the use of sodium acetate as one of the carriers of the reserve alkalinity.

The disclosure is a combined hyperosmolar solution for infusion comprising polyol, sodium chloride, potassium chloride, calcium chloride, magnesium chloride, sodium lactate and water for injections, and additionally comprising sodium acetate and L-malate, at the following component ratio by dry weight, wt. %:

| | |
|---|---|
| polyol | 4 - 7 |
| sodium chloride | 0,54 - 0,66 |
| potassium chloride | 0,027 - 0,033 |
| calcium chloride | 0,008 - 0,012 |
| magnesium chloride | 0,017 - 0,023 |

| | |
|---|---|
| sodium lactate | 1,35 - 1,65 |
| sodium acetate | 0,234 - 0,286 |
| L-malate | 0,120 - 0,145 |
| water for injections | remainder up to 100% |

In various embodiments, the present disclosure provides the combined hyperosmolar solution for infusion comprising sorbitol, xylitol, or a mixture thereof as a polyol.

One embodiment of the present disclosure provides the combined hyperosmolar solution for infusion comprising calcium chloride, magnesium chloride, sodium lactate, sodium acetate in the form of anhydrous salts or crystallohydrates.

In different embodiments, the present disclosure provides the combined hyperosmolar solution for infusion comprising L-malate in the form of L-malic acid or sodium salt of L-malic acid.

Another embodiment of the present disclosure provides the combined hyperosmolar solution for infusion comprising sorbitol, sodium chloride, potassium chloride, calcium chloride, magnesium chloride, sodium lactate, sodium acetate, L-malate and water for injections, at the following component ratio by dry weight, wt. %:

| | |
|---|---|
| sorbitol | 5 - 7 |
| sodium chloride | 0,54 - 0,66 |
| potassium chloride | 0,027 - 0,033 |
| calcium chloride | 0,008 - 0,012 |
| magnesium chloride | 0,017 - 0,023 |
| sodium lactate | 1,35 - 1,65 |
| sodium acetate | 0,234 - 0,286 |
| L-malate | 0,120 - 0,145 |
| water for injections | remainder up to 100% |

Yet another embodiment of the present disclosure provides the combined hyperosmolar solution for infusion comprising xylitol, sodium chloride, potassium chloride, calcium chloride, magnesium chloride, sodium lactate, sodium acetate, L-malate and water for injection, at the following component ratio by dry weight, wt. %:

| | |
|---|---|
| xylitol | 4 - 6 |
| sodium chloride | 0,54 - 0,66 |
| potassium chloride | 0,027 - 0,033 |
| calcium chloride | 0,008 - 0,012 |
| magnesium chloride | 0,017 - 0,023 |
| sodium lactate | 1,35 - 1,65 |
| sodium acetate | 0,234 - 0,286 |
| L-malate | 0,120 - 0,145 |
| water for injections | remainder up to 100% |

Another embodiment of the present disclosure provides the combined hyperosmolar solution for infusion with an osmolarity value in the range of 880-920 mOsm/L.

The main active pharmaceutical ingredients of the claimed solution for infusion are polyol, such as hexahydric alcohol sorbitol or pentahydric alcohol xylitol; reserve alkalinity carriers - sodium lactate, sodium acetate (alkalizing components); L-malate and electrolytes.

Reserve alkalinity carriers - are substances that, as a result of metabolism in the human body, form bicarbonate ions, thus replenishing the buffer capacity of the blood.

Since the known solution for infusion RHEOSORBILACT^{®} contains a sufficiently large amount of such a reserve alkalinity carrier as sodium lactate (19 mg/ml), it cannot be used for the treatment of patients with elevated blood level of lactate or disease that causes lactic acidosis (hypoxic conditions, cancer, cardiovascular disease, diabetes, etc.). Thus, the likelihood of developing possible side effects when using the known solution for infusion somewhat narrows the options for its use in the clinical practice.

To overcome the known disadvantages of the prototype, instead of introducing large amounts of sodium lactate into the solution for infusion, it was proposed to change this alkalizing component to a combination of 3 reserve alkalinity carriers, such as sodium lactate, sodium acetate and L-malate. This avoids the negative effects of overdose of the patient's body with lactate ion during infusion therapy using the prototype and makes the claimed combined hyperosmolar solution for infusion safer and more versatile in use, compared to the prototype. In this case, the qualitative and quantitative composition of the claimed solution for infusion is developed in such a way that the total osmolarity of the solution is maintained at an effective level close to 900 mOsmol/L ± 2%.

The claimed solution for infusion contains the reserve alkalinity carrier, namely sodium lactate, in concentration of 1,5% (133 mmol/L), which is 4 times higher than its content in the most common isoosmolar solutions (for example, in the RINGER'S LACTATE SOLUTION medicinal product-http://mozdocs.kiev.ua/likiview.php?id=13629), but less than in RHEOSORBILACT^{®} medicinal product (169,6 mmol/L). Sodium lactate helps to correct the blood plasma acid-base balance, as well as participates in the reactions of carbohydrate and energy metabolism, renews and stimulates the cell function of the reticuloendothelial system, liver and kidneys. Unlike the solutions for infusion containing sodium bicarbonate, such a reserve alkalinity carrier as sodium lactate has a neutral reaction. Sodium lactate is a slow-acting reserve alkalinity carrier. When sodium lactate is introduced into the blood stream, sodium, CO₂ and H₂O are released and form sodium bicarbonate, which leads to an increase in the blood alkaline reserve. Correction of metabolic acidosis with sodium lactate is slow (as sodium lactate is included in the metabolism) and does not cause sharp fluctuations in blood pH level. The effect of sodium lactate is manifested in 20-30 minutes after administration of the claimed solution for infusion.

Such a reserve alkalinity carrier as sodium acetate corrects well metabolic acidosis and is completely metabolized into an equivalent amount of sodium bicarbonate within 1,5 - 2 hours. Sodium acetate does not cause intracellular interstitial edema of the brain and increased aggregation of platelets and erythrocytes. It is important that sodium acetate has a high alkalizing effect with minimal consumption of O₂. Sodium acetate is metabolized mainly in the muscle tissue by acetyl-coenzyme A-synthetase and eventually converted to carbon monoxide and water. The alkalizing effect of acetate is manifested very quickly; the concentration of HCO₃⁻ ions increases within 15 minutes after the start of infusion of acetate ions; 90% of the administered amount of acetate ions is oxidized within a few minutes; between 60% and 80% of acetate ions are excreted as CO₂ through the lungs within 1 to 12 hours. The acetate ions metabolism does not change in patients with diabetes mellitus, and there are no changes in glucose and insulin concentrations. Acetate is a source of energy that supplies 209 kcal/mol. It also plays an important role in carbohydrate and lipid metabolism (acetate replaces fats as an oxidizing fuel without affecting glucose oxidation).

The inclusion of malate ions in the claimed solution for infusion allows enhancing the antihypoxant and detoxifying effects because malate is an energy substrate of the Krebs cycle, participating in the ornithine cycle of urea synthesis and in the binding of ammonia in muscles. The alkalizing effect of malate is much slower than that of acetate, which may be useful for achieving a long-term effect of the solution for infusion. The use of acetate and malate anions as the reserve alkalinity carriers allows minimizing the consumption of oxygen in the tissues required for the formation of bicarbonate ions.

Sorbitol is a hexahydric alcohol that is a white crystalline powder with sweet taste. Sorbitol is a source of energy with an insulin-independent metabolism (it is metabolized to fructose), which does not lead to an increase in blood glucose level. The sorbitol presence in the blood contributes to the normalization of carbohydrate and energy metabolism, stimulates the fatty acids oxidation by non-ketogenic metabolism and facilitates the use of ketone bodies in the Krebs cycle, which has a positive effect on improving the hepatocytes functional state. Sorbitol enhances intestinal motility due to the direct effect on the intestinal wall neuro-receptor apparatus and increased synthesis and secretion of villikinin, cholecystokinin and B vitamins. The maximum rate of sorbitol utilization is 0,25 g/kg body weight/h. The solution for infusion comprises sorbitol in an isotonic concentration of 6%.

According to one of the embodiments, the claimed solution for infusion, instead of sorbitol, contains such a pentahydric alcohol as xylitol that has a pronounced antiketogenic effect and is utilized in a non-insulin-dependent way, which makes it possible to safely use the solution for infusion by patients with diabetes mellitus and lactose intolerance. In addition, such a component as sorbitol is known for its effect on the body's digestive system (laxative effect, increased gas production, etc.). In cases when the patient has gastrointestinal disorders, it is more appropriate to use the claimed solution for infusion containing xylitol to avoid possible side effects.

Xylitol is a pentahydric alcohol that has the form of colorless crystals with sweet taste. It belongs to the intermediate products of carbohydrate metabolism in humans and animals. The end product of xylitol oxidation is carbon dioxide, which is released mainly with the exhaled air. When administered intravenously, xylitol is rapidly incorporated into the overall metabolism, and 80% is absorbed by the liver and accumulates as glycogen. Xylitol has low toxicity and good tolerability, does not cause a decrease in liver nucleotides (ATP, ADP, AMP), and is safe for administration to patients who do not tolerate fructose. Since xylitol is a good source of energy with insulin-independent metabolism, it acts antiketogenically and lipotropically. Therefore, the solutions for infusion containing xylitol are recommended to be used as means for parenteral nutrition of the patients, especially those who have undergone operations on the gastrointestinal tract. The maximum rate of utilization of xylitol is 0,25 g/kg body weight/hour. The solution for infusion comprises xylitol in isotonic concentration of 5%.

The presence of a balanced mixture of basic blood ions (Na⁺, K⁺, Ca⁺⁺, Mg⁺⁺, Cl⁻) in appropriate concentrations in the composition of the claimed solution for infusion makes the solution for infusion more physiological. Sodium chloride has a rehydrating effect, replenishes the deficiency of sodium and chlorine ions in various pathological conditions. Calcium is necessary for the process of transmitting nerve impulses, contraction of skeletal and smooth muscles, myocardial activity, bone formation, blood clotting. It reduces the permeability of cells and the vascular wall, prevents the development of inflammatory reactions, increases the body's resistance to infections. Magnesium is required for many biochemical reactions, glucose uptake, protein synthesis, nerve signaling, bone tissue formation, and more. Potassium restores water-electrolyte balance, has a negative chrono- and bathotropic effect, in high doses has a negative ino-, dromotropic and moderate diuretic effect. It participates in the process of transmitting nerve impulses, increases the content of acetylcholine and causes excitation of the sympathetic division of the autonomic nervous system.

Within the selected components and their quantitative ratio, the disclosure belongs to the group of multicomponent polyfunctional hyperosmolar solutions. The pharmacological action of the claimed solution for infusion is associated with the mutual potentiation of the action of its components, as well as the additional properties of the solution for infusion due to hyperosmolarity that is 3 times higher than the osmolarity of the blood plasma. After intravenous administration of the solution containing sorbitol, as well as the solution containing xylitol, the evacuation of fluid from the tissues into the blood stream increases, hemodynamics restores, microcirculation and rheological properties of the blood improve, blood viscosity reduces, platelet aggregation reduces, cardiac activity enhances, metabolic processes enhance, detoxification function of the liver improves. This allows promoting the claimed solution for infusion as means with hemodynamic, rheological, detoxification, antiketogenic, anti-shock, energy and alkalizing effect. The effective rheological and anti-shock action of the claimed solution for infusion enables infusion therapy of the patient using reduced amounts of the solution for infusion, i.e., the claimed solution for infusion can be used for the restrictive infusion therapy.

Therefore, the claimed combined hyperosmolar solution for infusion fully exhibits its pharmacological properties within the selected components and their quantitative ratio.

### BRIEF DESCRIPTION OF DRAWINGS

FIG. 1 - diagram of wavelet analysis of patient 1 before the infusion of the claimed solution for infusion.
FIG. 2 - diagram of wavelet analysis of patient 1 at 5 minutes after the infusion of the claimed solution for infusion.
FIG. 3 - diagram of wavelet analysis of patient 1 at 30 minutes after the infusion of the claimed solution for infusion.
FIG. 4 - diagram of wavelet analysis of patient 1 before the infusion of the comparative medicinal product.
FIG. 5 - diagram of wavelet analysis of patient 1 at 5 minutes after the infusion of the comparative medicinal product.
FIG. 6 - diagram of wavelet analysis of patient 1 at 30 minutes after the infusion of the comparative medicinal product.
FIG. 7 - diagram of wavelet analysis of patient 2 before the infusion of the claimed solution for infusion.
FIG. 8 - diagram of wavelet analysis of patient 2 at 5 minutes after the infusion of the claimed solution for infusion.
FIG. 9 - diagram of the wavelet analysis of patient 2 at 30 minutes after the infusion of the claimed solution for infusion.
FIG. 10 - diagram of wavelet analysis of patient 2 before the infusion of the comparative medicinal product.
FIG. 11 - diagram of the wavelet analysis of patient 2 at 5 minutes after the infusion of the comparative medicinal product.
FIG. 12 - diagram of the wavelet analysis of patient 2 at 30 minutes after the infusion of the comparative medicinal product.

### DESCRIPTION OF EMBODIMENTS

A technological scheme of the manufacturing of the claimed solution for infusion includes the following stages: weighing of the ingredients of appropriate quality, preparation of the solution, dilution of the solution to the required volume with water for injections, filtration, control of the physical and chemical parameters, bottling, sterilization, repeated control of the physical and chemical parameters, labeling, packaging.

The disclosure is illustrated by the following examples.

### Example 1.

The claimed combined hyperosmolar solution for infusion comprising sorbitol and L-malic acid is prepared as follows.

Preparation of the solution for infusion is carried out in an isolated, boxed room. 2/3 of water for injection is charged into the measuring mixer, and 60 g of sorbitol, 6 g of sodium chloride, 0,3 g of potassium chloride, 0,13 g of calcium chloride dihydrate, 0,43 g of magnesium chloride hexahydrate, 4,31 g of sodium acetate trihydrate, 1,34 g of L-malic acid are dissolved. Then 15 g of sodium lactate is added to the solution, the solution is diluted to the nominal volume (1 L) with water for injections and mixed to obtain the final solution for infusion.

The resulting solution for infusion then undergoes stages of filtration, packaging, sterilization, and quality control.

The resulting solution was filtered under a column of liquid to obtain a filtrate. Filters with a pore diameter of 1,2 µm (coarse purification) and 0,22 µm (fine purification) were used for filtration. The first portions of the filtrate were re-filtrated. The filtered solution was monitored for a lack of mechanical impurities, poured into 100-, 200-, or 400-ml bottles made of MTO or PSD grade glass for the blood, transfusion and infusion medicinal products, corked with stoppers of rubber mixture and enwrapped with aluminum caps or aluminum-plastic caps. The bottles with the solution were sterilized in a steam sterilizer by steam under a pressure of 1,1 Bar at a temperature of 121 °C for 30 minutes. The sterile solution was labeled and packaged after inspection for mechanical inclusions. Quality control of the solution for infusion was carried out in accordance with the requirements of the analytical and regulatory document regarding all qualitative and quantitative indicators.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 2.

The claimed combined hyperosmolar solution for infusion comprising sorbitol and sodium L-malate is prepared as follows.

Preparation of the solution for infusion is carried out in an isolated, boxed room. 2/3 of water for injection is charged into the measuring mixer, and 60 g of sorbitol, 6 g of sodium chloride, 0,3 g of potassium chloride, 0,13 g of calcium chloride dihydrate, 0,43 g of magnesium chloride hexahydrate, 4,31 g of sodium acetate trihydrate, 1,56 g of sodium L-malate are dissolved. Then 15 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 3.

The claimed combined hyperosmolar solution for infusion comprising sorbitol and L-malic acid is prepared as follows.

Preparation of the solution for infusion is carried out in an isolated, boxed room. 2/3 of water for injection is charged into the measuring mixer, and 50 g of sorbitol, 5,4 g of sodium chloride, 0,27 g of potassium chloride, 0,11 g of calcium chloride dihydrate, 0,36 g of magnesium chloride hexahydrate, 3,88 g of sodium acetate trihydrate, 1,22 g of L-malic acid are dissolved. Then 13,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 4.

The claimed combined hyperosmolar solution for infusion comprising sorbitol and sodium L-malate is prepared as follows.

Preparation of the solution for infusion is carried out in an isolated, boxed room. 2/3 of water for injection is charged into the measuring mixer, and 50 g of sorbitol, 5,4 g of sodium chloride, 0,27 g of potassium chloride, 0,11 g of calcium chloride dihydrate, 0,36 g of magnesium chloride hexahydrate, 3,88 g of sodium acetate trihydrate, 1,42 g of sodium L-malate are dissolved. Then 13,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 5.

The claimed combined hyperosmolar solution for infusion comprising sorbitol and L-malic acid is prepared as follows.

Preparation of the solution for infusion is carried out in an isolated, boxed room. 2/3 of water for injection is charged into the measuring mixer, and 70 g of sorbitol, 7 g of sodium chloride, 0,33 g of potassium chloride, 0,16 g of calcium chloride dihydrate, 0,49 g of magnesium chloride hexahydrate, 4,74 g of sodium acetate trihydrate, 1,47 g of L-malic acid are dissolved. Then 16,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 6.

The claimed combined hyperosmolar solution for infusion comprising sorbitol and sodium L-malate is prepared as follows.

Preparation of the solution for infusion is carried out in an isolated, boxed room. 2/3 of water for injection is charged into the measuring mixer, and 70 g of sorbitol, 7 g of sodium chloride, 0,33 g of potassium chloride, 0,16 g of calcium chloride dihydrate, 0,49 g of magnesium chloride hexahydrate, 4,74 g of sodium acetate trihydrate, 1,71 g of sodium L-malate are dissolved. Then 16,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 7.

The claimed combined hyperosmolar solution for infusion comprising xylitol and L-malic acid is prepared as follows.

Preparation of the solution for infusion is carried out in an isolated, boxed room. 2/3 of water for injection is charged into the measuring mixer, and 50 g of xylitol, 6 g of sodium chloride, 0,3 g of potassium chloride, 0,13 g of calcium chloride dihydrate, 0,43 g of magnesium chloride hexahydrate, 4,31 g of sodium acetate trihydrate, 1,34 g of L-malic acid are dissolved. Then 15 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 8.

The claimed combined hyperosmolar solution for infusion comprising xylitol and sodium L-malate is prepared as follows.

Preparation of the solution for infusion is carried out in an isolated, boxed room. 2/3 of water for injection is charged into the measuring mixer, and 50 g of xylitol, 6 g of sodium chloride, 0,3 g of potassium chloride, 0,13 g of calcium chloride dihydrate, 0,43 g of magnesium chloride hexahydrate, 4,31 g of sodium acetate trihydrate, 1,56 g of sodium L-malate are dissolved. Then 15 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 9.

The claimed combined hyperosmolar solution for infusion comprising xylitol and L-malic acid is prepared as follows.

Preparation of the solution for infusion is carried out in an isolated, boxed room. 2/3 of water for injection is charged into the measuring mixer, and 40 g of xylitol, 5,4 g of sodium chloride, 0,27 g of potassium chloride, 0,11 g of calcium chloride dihydrate, 0,36 g of magnesium chloride hexahydrate, 3,88 g of sodium acetate trihydrate, 1,22 g of L-malic acid are dissolved. Then 13,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 10.

The claimed combined hyperosmolar solution for infusion comprising xylitol and sodium L-malate is prepared as follows.

Preparation of the solution for infusion is carried out in an isolated, boxed room. 2/3 of water for injection is charged into the measuring mixer, and 40 g of xylitol, 5,4 g of sodium chloride, 0,27 g of potassium chloride, 0,11 g of calcium chloride dihydrate, 0,36 g of magnesium chloride hexahydrate, 3,88 g of sodium acetate trihydrate, 1,42 g of sodium L-malate are dissolved. Then 13,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 11.

The claimed combined hyperosmolar solution for infusion comprising xylitol and L-malic acid is prepared as follows.

Preparation of the solution for infusion is carried out in an isolated, boxed room. 2/3 of water for injections is charged into the measuring mixer, and 60 g of xylitol, 7 g of sodium chloride, 0,33 g of potassium chloride, 0,16 g of calcium chloride dihydrate, 0,49 g of magnesium chloride hexahydrate, 4,74 g of sodium acetate trihydrate, 1,47 g of L-malic acid are dissolved. Then 16,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 12.

The claimed combined hyperosmolar solution for infusion comprising xylitol and sodium L-malate is prepared as follows.

Preparation of the solution for infusion is carried out in an isolated, boxed room. 2/3 of water for injections is charged into the measuring mixer, and 60 g of xylitol, 7 g of sodium chloride, 0,33 g of potassium chloride, 0,16 g of calcium chloride dihydrate, 0,49 sodium L-malate are dissolved. Then 16,5 g of sodium lactate is added to the solution, the solution is diluted to the nominal volume (1 L) with water for injections and mixed to obtain the final solution for infusion.

The resulting solution for infusion then undergoes the stages of filtration, packaging, sterilization, and quality control as described in Example 1.

The resulting solution is a colorless, clear, odorless liquid. The stability of the solution is tested under sterilization conditions, as well as during storage. It was found that the physical and chemical properties of the solution met the requirements of the analytical and regulatory document, i.e., the choice of the components and technology of the solution production fully met the task set in the disclosure.

### Example 13.

The study of the claimed solution for infusion for specific activity was performed on a rabbit model of toxic hepatitis caused by subcutaneous injections of carbon tetrachloride for 7 days. The solution for infusion prepared according to Example 8 was administered at a dose of 10 ml/kg body weight for 14 days, starting from the first day of the study. The results showed that during the first seven days of the study statistically significant (<0,05) increase in ALA (0,43+0,04 to 1,39+0,03 mmol/L×h), AST (0,30+0,02 to 1,09+0,02 mmol/L×h) and bilirubin (11,68+0,95 to 21,60+1,55 µmol/L) was observed in all the animals on the background of the introduction of carbon tetrachloride. At the end of the study, on the 14th day, the activity of these indicators was statistically significantly reduced, but did not reach the initial level (ALT to 0,71+0,02 mmol/L×h, AST to 0,48+0,02 mmol/L×h, bilirubin to 16,15+1,30 µmol/L). Thus, in the study on the rabbit model of toxic hepatitis, it was shown that the claimed solution for infusion at a dose of 10 ml/kg body weight exhibits significant detoxifying properties.

### Example 14.

The results of the experimental study of chronic toxicity of the solution prepared according to Example 8 in rabbits (when administering intravenously at a dose of 10 ml/kg body weight for 30 days) confirmed that the medicinal product has no cumulative properties. Administration of the solution to the rabbits does not cause pathological changes of hemogram, hemostasis, electrolyte concentration, biochemical blood parameters, as well as urine parameters of the animals. Pathomorphological studies indicate that after repeated intravenous injections of the claimed solution for infusion to the rabbits, the internal organs of the study animals retained the normal structure without specific pathological changes and do not differ histologically from the organs of the control animals.

### Example 15,

During the experimental study of acute toxicity in the animals, it was found that the claimed solution for infusion prepared according to Example 8 belongs to a class of relatively safe substances when administered intraperitoneally to white mice and white rats. The death of the animals and symptoms of intoxication were not observed after a single injection of the solution to white mice at a dose of 50 ml/kg body weight and white rats at a dose of 45 ml/kg body weight. Repeated divided retroperitoneal administration of the solution to white mice and white rats at the maximum possible excessive dose (180 ml/kg body weight for mice and 100 ml/kg for rats) did not cause death of the animals.

### Example 16.

Comparative study of the effect of the claimed solution for infusion on the microcirculation level.

The study was performed using Laser Doppler Flowmetry (LDF) method with wavelet analysis. The results were recorded three times: before infusion (baseline recording), at 5 and 30 minutes after the infusion. The occlusion test was performed by applying a cuff for 3 minutes and determining the capillary blood flow reserve (CBFR).

The assessment of the microcirculation level in the subjects was based on the analysis of the following parameters.

Microcirculation score (MS) characterizes tissue blood flow and is a level of perfusion (blood flow) per unit volume of tissue per unit time. It reflects the volume content of erythrocytes in the capillary blood and the number of simultaneously functioning capillaries in studied area.

Capillary blood flow reserve (CBFR) reflects the reserve capacity of the microcirculatory tract in terms of increase of the microcirculation rate during reactive post-occlusion hyperemia.

An open prospective study was conducted. The study involved 2 patients (1 male and 1 female). The infusion of the solutions was performed in 2 stages. The microcirculatory effect of two solutions for infusion, namely the claimed solution for infusion, 200 ml (composition: 100 ml of the solution contained xylitol 5 g; sodium chloride 0,6 g; potassium chloride 0,03 g; calcium chloride 0,01 g; magnesium chloride 0,02 g; sodium lactate 1,5 g; sodium acetate 0,26 g; L-malic acid 0,134 g), and the comparative medicinal product, such as RHEOSORBILACT^{®} solution for infusion, 200 ml (composition: 100 ml of the solution contained sorbitol 6 g; sodium lactate 1,9 g; sodium chloride 0,6 g; calcium chloride 0,01 g; potassium chloride 0,03 g; magnesium chloride 0,02 g) were compared. Determination of the microcirculation score (MS), as well as capillary blood flow reserve (CBFR) using an occlusive test, was performed before the infusion, at 5 and 30 minutes after the infusion.

Patient 1 was infused with the claimed solution. During the baseline recording the value of MS = 3,27 perfusion units (pf.un.) was recorded; at 5 minutes after the infusion MS increased to 6,14 pf.un; at 30 minutes after the infusion MS increased to 9,51 pf.un. Thus, during the infusion of the claimed solution, patient 1 showed an increase in MS from 3,14 to 6,14 at 5 minutes after the infusion and a significant steady increase in MS to 9,51 at 30 minutes after the infusion. 5 min after the infusion of the claimed solution, there was a decrease in the capillary blood flow reserve from 187% to 109% and there was a further decrease in RCBF to 103,16% at 30 minutes after the infusion, indicating the opening of a portion of the reserve capillaries due to the infusion of the claimed solution.

On day 2, patient 1 was infused with the comparative medicinal product. During the baseline recording the value of MS = 6,45 pf.un. was obtained, at 5 min after the infusion MS showed a slight increase to 8,13 pf.un., and at 30 min the score remained almost stable, such as MS = 8,23 pf.un., which is much lower than the scores for the claimed solution for infusion; and the comparative medicinal product did not show a stable residual effect. Also, there was a decrease in RCBF from 156% of the baseline record to 112% at 5 min after the infusion, as well as a slight decrease in RCBF to 99% at 30 min after the infusion.

At examination of patient 2, on the first day the claimed solution was infused. During the baseline recording the value of MS = 8,06 pf.un. was obtained, at 5 min after the infusion MS increased to the value of 8,24 pf.un., and at 30 min after the infusion MS increased to the value of 10,56 pf.un. Thus, there was a slight increase in MS from 8,06 to 8,24 pf.un., as well as a significant increase in MS to 10,56 pf.un. at 30 min after the infusion. There was a decrease in the value of RCBF from 197% to 115% at 5 min after the infusion, and to 106% at 30 min after the infusion.

At 2^{nd} day, patient 2 was infused with the comparative medicinal product. During the baseline recording, the value of MS = 8,52 pf.un. was obtained, at 5 min after the infusion, the MS increased to the value of 8,56 pf.un., and at 30 min after the infusion, the MS increased to the value of 9,01 pf.un. That is, there was a slight increase in the value of MS from 8,52 to 8,56 pf.un. at 5 min after the infusion, as well as a slight increase in MS to 9,01 pf.un. at 30 min after the infusion. At the same time, the value of the RCBF decreased from 141% to 91% at 5 min after the infusion, and to 80% at 30 min after the infusion. The results of the study are shown in Table 1.

**Table 1**

| The results of the studies of the effect of the solution for infusion and the comparative medicinal product on the microcirculation scores and the capillary blood flow reserve | | | | | | |
|---|---|---|---|---|---|---|
| | Claimed solution for infusion | | | Comparative medicinal product | | |
| | MS baseline | 5 min | 30 min | MS baseline | 5 min | 30 min |
| Patient 1 | 3,27 | 6,14 | 9,51 | 6,45 | 8,13 | 8,23 |
| | RCBF baseline | | | RCBF baseline | | |
| | 187,80 | 109,61 | 103,16 | 156,47 | 111,97 | 99,20 |
| | MS baseline | | | MS baseline | | |
| Patient 2 | 8,06 | 8,24 | 10,56 | 8,52 | 8,56 | 9,01 |
| | RCBF baseline | | | RCBF baseline | | |
| | 197,04 | 114,68 | 106,44 | 141,64 | 90,89 | 80,35 |

Thus, the study showed a significant increase in microcirculation when using the claimed solution for infusion in both examined patients at 5 min after the infusion, as well as a stable and long-lasting effect at 30 min after the infusion, due to relaxation of the precapillary sphincters. This is probably due to the activation of A3 adenosine receptors by the claimed combined solution for infusion containing polyol, lactate, L-malate and acetate ions. There was a decrease in the RCBF at 5 min and 30 min after the infusion, which indicates the opening of a portion of the reserve capillaries due to the infusion of the claimed solution.

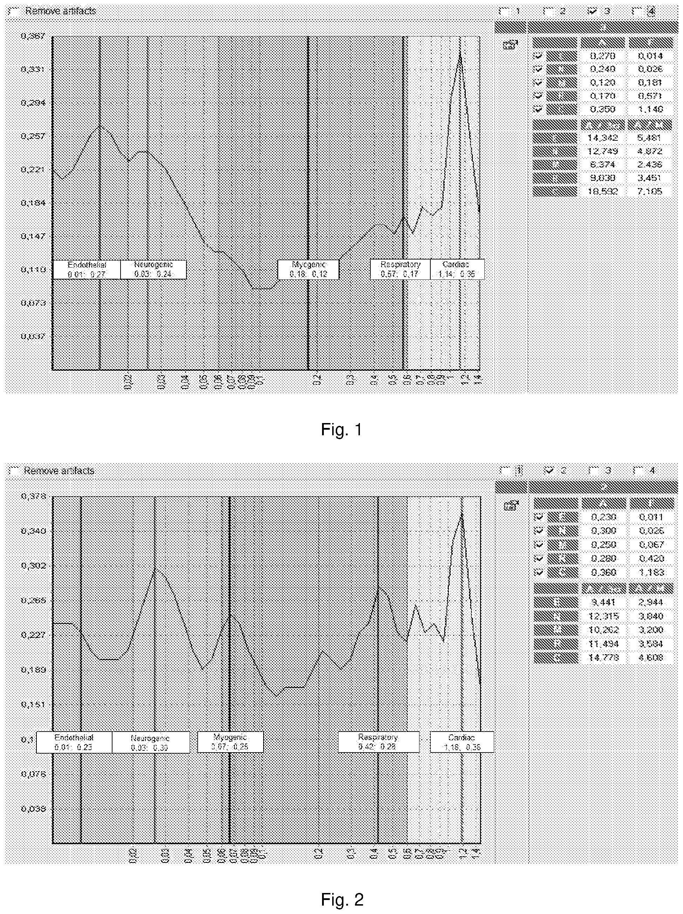

As shown in Figures 1-3, use of the claimed solution for infusion improves the microcirculation due to the relaxation of the precapillary sphincters provided by a decrease in the capillaries myotonus. Also, there was an increase in the maximum amplitude of respiratory fluxmotions.

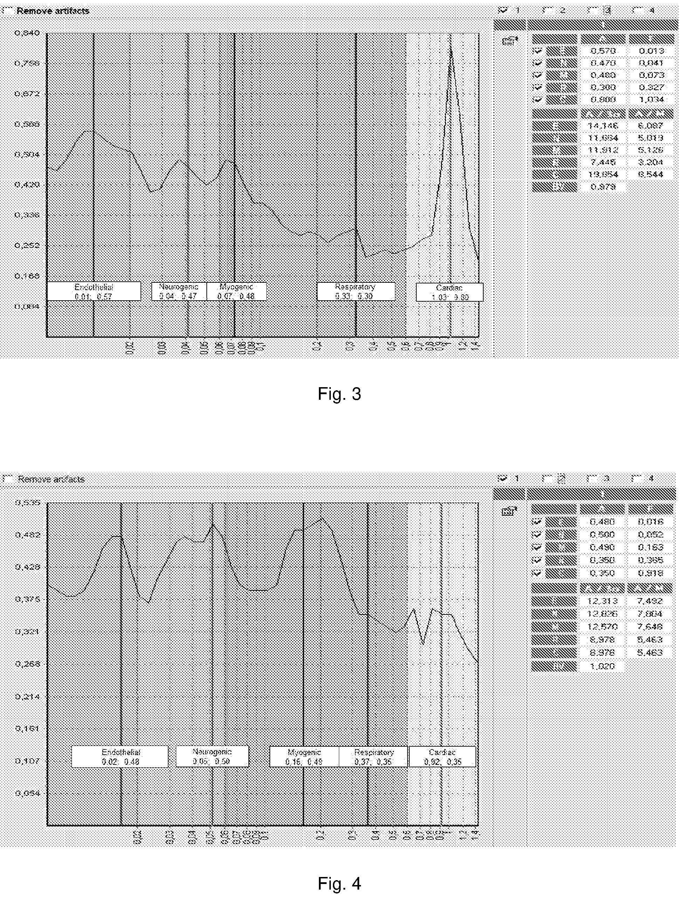

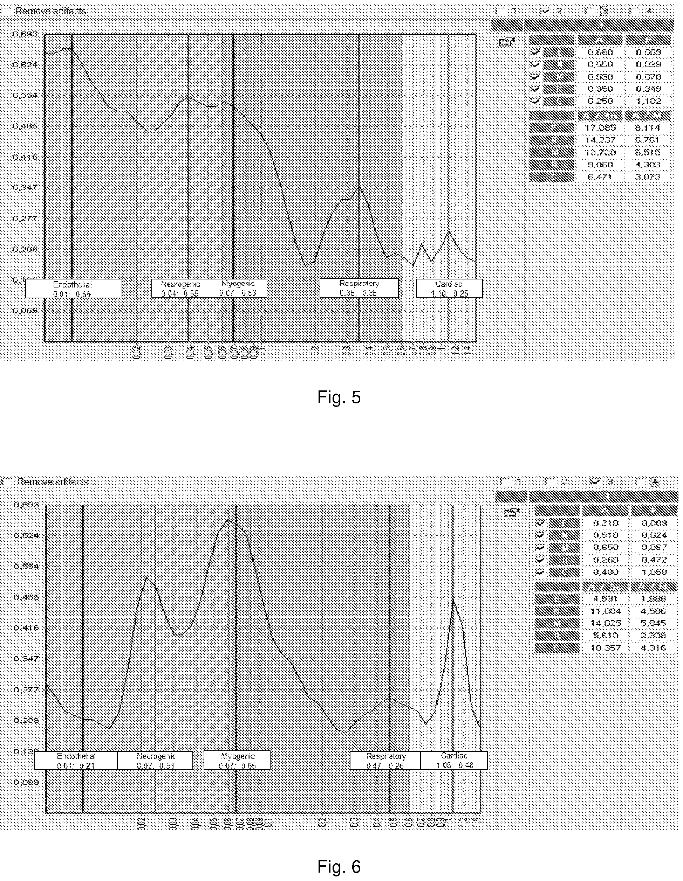

As shown in Figures 4-6, use of the comparative medicinal product slightly improves the microcirculation due to increased exposure to the endothelial component, and a decrease in capillary myotonus, which causes relaxation of the precapillary sphincters.

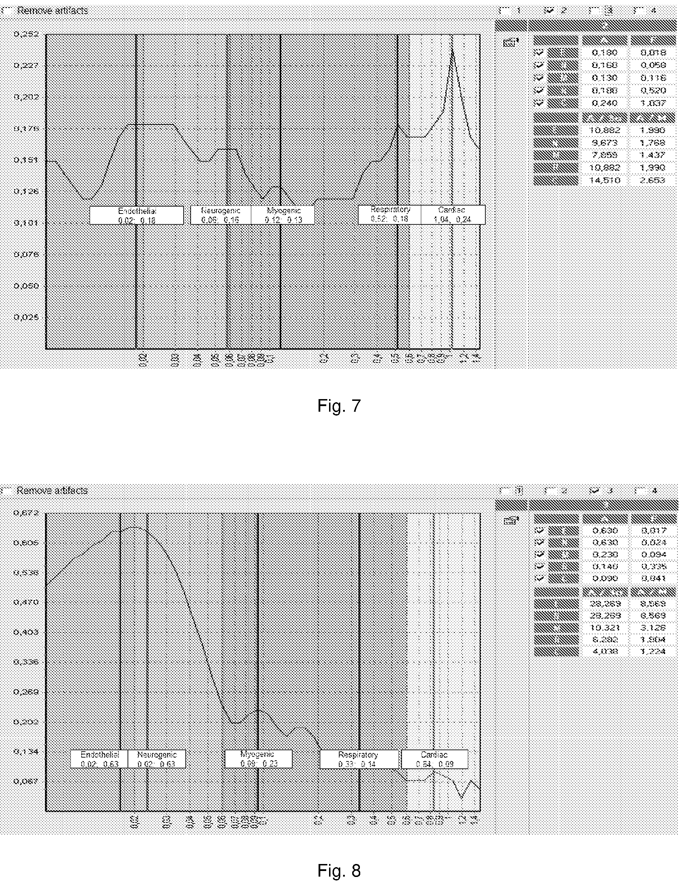

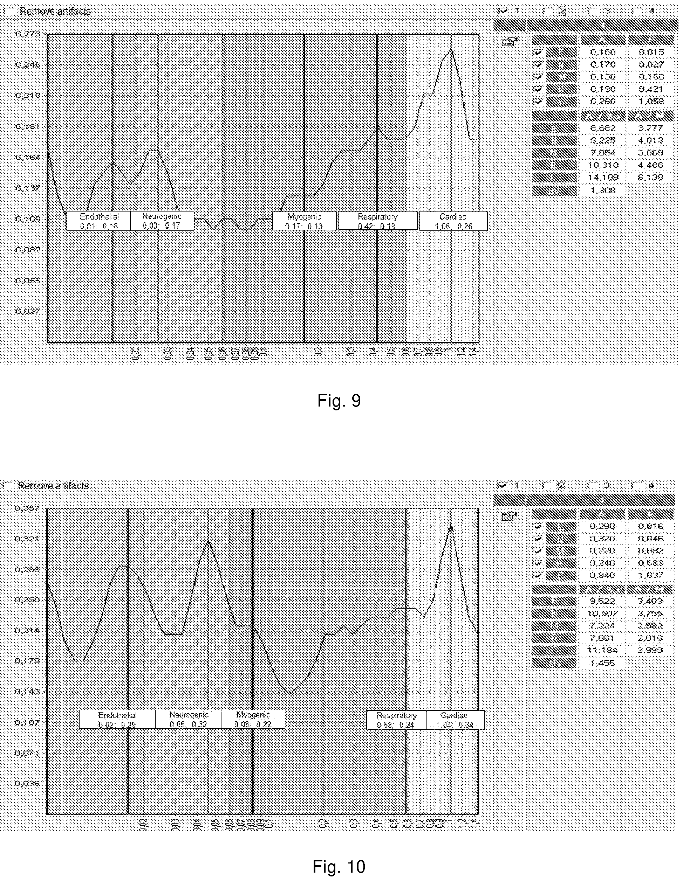

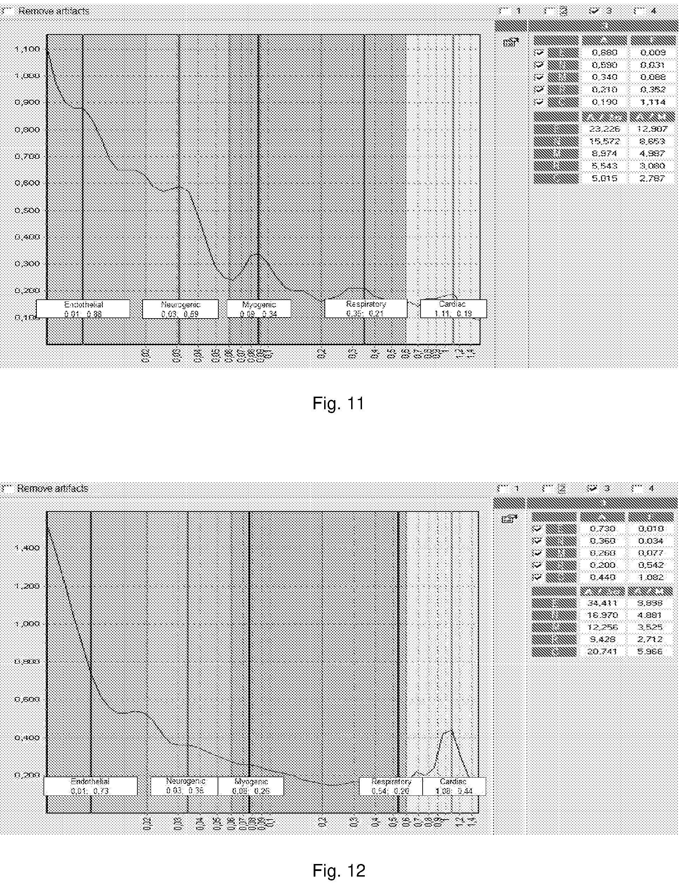

As shown in Figures 7-12, in contrast to the comparative medicinal product, use of the claimed solution for infusion steadily increases the endothelial component of the microcirculation regulation, as well as decreases capillary neurotonicity, which improves microcirculation.

### Example 17.

To prove the efficacy and safety of the different compositions of the claimed solution for infusion, a series of three clinical trials with the same design and the total number of included patients (111) was conducted.

Randomized open-label parallel comparative studies, which confirm the efficacy and safety of the different compositions of the claimed solution for infusion in comparison with the known RHEOSORBILACT^{®} medicinal product, were performed.

The study included patients aged 18 to 60 years with the following pathological conditions:
- traumatic, operative, hemolytic, toxic or burn shock;
- burn disease;
- acute bleeding;
- infectious diseases accompanied by intoxication;
- exacerbation of hepatitis;
- sepsis;
- thrombophlebitis.

According to the results of the laboratory parameters, all the above-mentioned conditions were accompanied by metabolic acidosis of varying severity, electrolyte and rheological imbalance of the blood. Depending on the severity of the pathology, hemodynamic and microcirculatory abnormalities were observed. Patients with excessively high lactate levels were not included in the study.

All the patients received standard treatment of the above pathologies in accordance with the treatment protocols of the Ministry of Health of Ukraine. Different compositions of the claimed solution for infusion or the RHEOSORBILACT^{®} comparative medicinal product were used as hyperosmolar solutions.

To assess the effectiveness, during the entire course of treatment with the claimed solution for infusion, the following parameters were measured: patient's general condition, subjective signs of the pathology, general blood test, acid-base balance, coagulogram, ionic and gaseous composition of the blood, arterial and venous pressure, dynamics of diuresis.

Study No. 1 included 45 patients, who were randomized to 3 groups of 15 subjects. Group 1 was treated with the claimed solution for infusion of the following composition (wt.%) as the hyperosmolar solution:

| Sorbitol | 5,0 |
|---|---|
| Sodium chloride | 0,54 |
| Potassium chloride | 0,027 |
| Calcium chloride | 0,008 |
| Magnesium chloride | 0,017 |
| Sodium lactate | 1,35 |
| Sodium acetate | 0,234 |
| L-malic acid | 0,122 |
| Water for injections | remainder up to 100% |

Group 2 was treated with the claimed solution for infusion of the following composition (wt.%) as the hyperosmolar solution:

| Xylitol | 4,0 |
|---|---|
| Sodium chloride | 0,54 |
| Potassium chloride | 0,027 |
| Calcium chloride | 0,008 |
| Magnesium chloride | 0,017 |
| Sodium lactate | 1,35 |
| Sodium acetate | 0,234 |
| L-malic acid | 0,122 |
| Water for injections | remainder up to 100% |

Group 3 was treated with the comparative medicinal product as the hyperosmolar solution.

Study No. 2 included 30 patients, who were randomized to 3 groups of 10 subjects.

Group 1 was treated with the claimed solution for infusion of the following composition (wt.%) as the hyperosmolar solution:

| Sorbitol | 6,0 |
|---|---|
| Sodium chloride | 0,6 |
| Potassium chloride | 0,03 |
| Calcium chloride | 0,01 |
| Magnesium chloride | 0,02 |
| Sodium lactate | 1,5 |
| Sodium acetate | 0,26 |
| L-malic acid | 0,134 |
| Water for injections | remainder up to 100% |

Group 2 was treated with the claimed solution for infusion of the following composition (wt.%) as the hyperosmolar solution:

| Xylitol | 5,0 |
|---|---|
| Sodium chloride | 0,6 |
| Potassium chloride | 0,03 |
| Calcium chloride | 0,01 |
| Magnesium chloride | 0,02 |
| Sodium lactate | 1,5 |
| Sodium acetate | 0,26 |
| L-malic acid | 0,134 |
| Water for injections | remainder up to 100% |

Group 3 was treated with the comparative medicinal product as the hyperosmolar solution.

Study No. 3 included 36 patients, who were randomized to 3 groups of 12 subjects.

Group 1 was treated with the claimed solution for infusion of the following composition (wt.%) as the hyperosmolar solution:

| Sorbitol | 7,0 |
|---|---|
| Sodium chloride | 0,66 |
| Potassium chloride | 0,033 |
| Calcium chloride | 0,012 |
| Magnesium chloride | 0,023 |
| Sodium lactate | 1,65 |
| Sodium acetate | 0,286 |
| L-malic acid | 0,147 |
| Water for injections | remainder up to 100% |

Group 2 was treated with the claimed solution for infusion of the following composition (wt.%) as the hyperosmolar solution:

| Xylitol | 6,0 |
|---|---|
| Sodium chloride | 0,66 |
| Potassium chloride | 0,033 |
| Calcium chloride | 0,012 |
| Magnesium chloride | 0,023 |
| Sodium lactate | 1,65 |
| Sodium acetate | 0,286 |
| L-malic acid | 0,147 |
| Water for injections | remainder up to 100% |

Group 3 was treated with the comparative medicinal product as the hyperosmolar solution.

According to three studies, the following results were recorded and analyzed. The results of the clinical and laboratory studies indicate that the subjective complaints of the patients in Groups 1 and 2 disappeared earlier than in Group 3. Thus, in groups of the patients who received the claimed solution for infusion, body temperature was normalized, general weakness disappeared, diuresis was normalized, and appetite was improved 2-3 days earlier than in the patients of comparative groups.

It is noted that hemodynamic parameters (including the microcirculation) stabilized on average at 5-6 hour after the start of a combined therapy with the solutions in Groups 1 and Groups 2, and at 6-8 hour after the start of a combined therapy with the comparative medicinal product. Hematological parameters, in particular the level of erythrocytes, hemoglobin, hematocrit, and blood diastase, approached the baseline values at 24-48 hour after the start of the treatment in all the groups. Leukocyte counts, leukocyte formula and ESR in patients of Groups 1 and Groups 2 were normalized 2-3 days earlier than in patients of comparative groups.

In addition, in Groups 2 glycemic parameters improved. This can be explained by the presence of xylitol in the solutions for Groups 2, which, unlike sorbitol, is metabolized without insulin and is not transformed into fructose, and therefore can be used freely even for patients suffering from diabetes and fructose intolerance.

Before the start of the use of the studied hyperosmolar solutions, all the patients were suffering from metabolic acidosis of varying severity. During the treatment with the solutions, an alkalizing effect was observed in all the groups, but it was more pronounced for the patients of Groups 1 and Groups 2. Normalization of the acid-base status occurred at 36 hours after the use of the solutions. For patients of Group 3 normalization of acid-base status occurred at 48-72 hours after the use of the comparative medicinal product. This can be explained by the presence of acetate in the composition of the solutions of Groups 1 and 2, which is rapidly incorporated into the metabolism and releases HCO₃⁻ions within 15 minutes after administration to the patient. The rate of normalization of the acid-base parameters of the blood when using the claimed solution for infusion in different concentrations is directly proportional to the concentration of the reserve alkalinity carriers in the study medicinal products.

When using the claimed solution for injection and the comparative medicinal product, no side effects were observed throughout the study period.

Therefore, we can conclude that the use of the claimed solution for infusion in various combinations in the combined treatment of the patients with the above-mentioned pathologies has a positive effect on the regression rate of the clinical manifestations, normalization of blood acid-base balance, hemodynamics and microcirculation scores, and normalization of hemogram.

Thus, the use of the claimed combined hyperosmolar solution for infusion allows to achieve following result: the claimed combined hyperosmolar solution for infusion is effective and safe to use, and can be used for the treatment of a wide range of diseases, such as microcirculation and peripheral blood disorders; can be used to treat different groups of the population, including patients with diabetes and fructose intolerance; can be characterized by a faster onset of alkalizing action, reduced risk of overdose of the patient with lactate ion; can be used in smaller quantities, i.e., suitable for restrictive infusion therapy and, due to the combination of components and their quantitative ratio, has a multifunctional effect, namely: rheological, detoxification and anti-shock.

## Claims

1. Combined hyperosmolar solution for infusion comprising polyol, sodium chloride, potassium chloride, calcium chloride, magnesium chloride, sodium lactate and water for injection, **characterized in that** it further comprises sodium acetate and L-malate, at the following component ratio by dry weight, wt. %:
| | |
|---|---|
| polyol | 4-7 |
| sodium chloride | 0,54 - 0,66 |
| potassium chloride | 0,027 - 0,033 |
| calcium chloride | 0,008 - 0,012 |
| magnesium chloride | 0,017 - 0,023 |
| sodium lactate | 1,35 - 1,65 |
| sodium acetate | 0,234 - 0,286 |
| L-malate | 0,120 - 0,145 |
| water for injections | remainder up to 100% |

2. Combined hyperosmolar solution for infusion according to claim 1, **characterized in that** it comprises sorbitol, xylitol or a mixture thereof as a polyol.

3. Combined hyperosmolar solution for infusion according to any of claims 1 to 2, **characterized in that** it comprises calcium chloride, magnesium chloride, sodium lactate, sodium acetate in the form of anhydrous salts or crystallohydrates.

4. Combined hyperosmolar solution for infusion according to any of claims 1-3, **characterized in that** it comprises L-malate in the form of L-malic acid or sodium salt of L-malic acid.

5. Combined hyperosmolar solution for infusion according to any of claims 1-4, **characterized in that** it comprises sorbitol, sodium chloride, potassium chloride, calcium chloride, magnesium chloride, sodium lactate, sodium acetate, L-malate and water for injection, at the following component ratio by dry weight, wt. %:
| | |
|---|---|
| sorbitol | 5 - 7 |
| sodium chloride | 0,54 - 0,66 |
| potassium chloride | 0,027 - 0,033 |
| calcium chloride | 0,008 - 0,012 |
| magnesium chloride | 0,017 - 0,023 |
| sodium lactate | 1,35 - 1,65 |
| sodium acetate | 0,234 - 0,286 |
| L-malate | 0,120 - 0,145 |
| water for injections | remainder up to 100% |

6. Combined hyperosmolar solution for infusion according to any of claims 1-4, **characterized in that** it comprises xylitol, sodium chloride, potassium chloride, calcium chloride, magnesium chloride, sodium lactate, sodium acetate, L-malate and water for injection, at the following component ratio by dry weight, wt. %:
| | |
|---|---|
| xylitol | 4 - 6 |
| sodium chloride | 0,54 - 0,66 |
| potassium chloride | 0,027 - 0,033 |
| calcium chloride | 0,008 - 0,012 |
| magnesium chloride | 0,017 - 0,023 |
| sodium lactate | 1,35 - 1,65 |
| sodium acetate | 0,234 - 0,286 |
| L-malate | 0,120 - 0,145 |
| water for injections | remainder up to 100% |

## Patentansprüche

1. Kombinierte hyperosmolare Infusionslösung enthaltend Polyol, Natriumchlorid, Kaliumchlorid, Calciumchlorid, Magnesiumchlorid, Natriumlactat und Wasser zur Injektionskontrolle, **dadurch gekennzeichnet, dass** sie ferner Natriumacetat und L-Malat umfasst, mit folgendem Komponentenverhältnis nach Trockengewicht, Gew.-%:
| | |
|---|---|
| polyol | 4 - 7 |
| Natriumchlorid | 0,54 - 0,66 |
| Kaliumchlorid | 0,027 - 0,033 |
| Calciumchlorid | 0,008 - 0,012 |
| Magnesiumchlorid | 0,017 - 0,023 |
| Natriumlaktat | 1,35 - 1,65 |
| Natriumacetat | 0,234 - 0,286 |
| L-Malat | 0,120 - 0,145 |
| Wasser für Injektionszwecke | Rest bis zu 100% |

2. Kombinierte hyperosmolare Infusionslösung nach Anspruch 1, **dadurch gekennzeichnet, dass** sie Sorbit, Xylit oder ein Gemisch davon als Polyol umfasst.

3. Kombinierte hyperosmolare Infusionslösung nach einem der Ansprüche 1 bis 2, **dadurch gekennzeichnet, dass** sie Calciumchlorid, Magnesiumchlorid, Natriumlactat, Natriumacetat in Form von wasserfreien Salzen oder Kristallhydraten umfasst.

4. Kombinierte hyperosmolare Infusionslösung nach einem der Ansprüche 1-3, **dadurch gekennzeichnet, dass** sie L-Malat in Form von L-Äpfelsäure oder Natriumsalz von L-Äpfelsäure umfasst.

5. Kombinierte hyperosmolare Infusionslösung nach einem der Ansprüche 1-4, **dadurch gekennzeichnet, dass** sie Sorbit, Natriumchlorid, Kaliumchlorid, Calciumchlorid, Magnesiumchlorid, Natriumlactat, Natriumacetat, L-Malat und Wasser für Injektionszwecke umfasst, mit folgendem Komponentenverhältnis nach Trockengewicht, Gew.-%:
| | |
|---|---|
| Sorbit | 5 - 7 |
| Natriumchlorid | 0,54 - 0,66 |
| Kaliumchlorid | 0,027 - 0,033 |
| Calciumchlorid | 0,008 - 0,012 |
| Magnesiumchlorid | 0,017 - 0,023 |
| Natriumlaktat | 1,35 - 1,65 |
| Natriumacetat | 0,234 - 0,286 |
| L-Malat | 0,120 - 0,145 |
| Wasser für Injektionszwecke | Rest bis zu 100% |

6. Kombinierte hyperosmolare Infusionslösung nach einem der Ansprüche 1-4, **dadurch gekennzeichnet, dass** sie Xylit, Natriumchlorid, Kaliumchlorid, Calciumchlorid, Magnesiumchlorid, Natriumlactat, Natriumacetat, L-Malat und Wasser zur Injektion umfasst, mit folgendem Komponentenverhältnis nach Trockengewicht, Gew.-%:
| | |
|---|---|
| Xylitol | 4 - 6 |
| Natriumchlorid | 0,54 - 0,66 |
| Kaliumchlorid | 0,027 - 0,033 |
| Calciumchlorid | 0,008 - 0,012 |
| Magnesiumchlorid | 0,017 - 0,023 |
| Natriumlaktat | 1,35 - 1,65 |
| Natriumacetat | 0,234 - 0,286 |
| L-Malat | 0,120 - 0,145 |
| Wasser für Injektionszwecke | Rest bis zu 100% |

## Revendications

1. Solution hyperosmolaire combinée pour perfusion comprenant du polyol, du chlorure de sodium, du chlorure de potassium, du chlorure de calcium, du chlorure de magnésium, du lactate de sodium et de l'eau pour préparations injectables, **caractérisée en ce qu'**elle comprend en outre de l'acétate de sodium et du L-malate, au rapport de composants suivant en poids sec, % en poids :
| | |
|---|---|
| Polyol | 4 - 7 |
| chlorure de sodium | 0,54 - 0,66 |
| chlorure de potassium | 0,027 - 0,033 |
| chlorure de calcium | 0,008 - 0,012 |
| chlorure de magnésium | 0,017 - 0,023 |
| lactate de sodium | 1,35 - 1,65 |
| acétate de sodium | 0,234 - 0,286 |
| L-malate | 0,120 - 0,145 |
| Eau pour préparations injectables | reste jusqu'à 100% |

2. Solution hyperosmolaire combinée pour perfusion selon la revendication 1, **caractérisée en ce qu'**elle comprend du sorbitol, du xylitol ou un mélange de ceux-ci sous forme de polyol.

3. Solution hyperosmolaire combinée pour perfusion selon l'une quelconque des revendications 1 à 2, **caractérisée en ce qu'**elle comprend du chlorure de calcium, du chlorure de magnésium, du lactate de sodium, de l'acétate de sodium sous forme de sels anhydres ou d'hydrates cristallo.

4. Solution hyperosmolaire combinée pour perfusion selon l'une quelconque des revendications 1 à 3, **caractérisée en ce qu'**elle comprend du L-malate sous forme d'acide L-malique ou du sel de sodium de l'acide L-malique.

5. Solution hyperosmolaire combinée pour perfusion selon l'une quelconque des revendications 1 à 4, **caractérisée en ce qu'**elle comprend du sorbitol, du chlorure de sodium, du chlorure de potassium, du chlorure de calcium, du chlorure de magnésium, du lactate de sodium, de l'acétate de sodium, du L-malate et de l'eau pour préparations injectables, au rapport de composants suivant en poids sec, % en poids :
| | |
|---|---|
| sorbitol | 5 - 7 |
| chlorure de sodium | 0,54 - 0,66 |
| chlorure de potassium | 0,027 - 0,033 |
| chlorure de calcium | 0,008 - 0,012 |
| chlorure de magnésium | 0,017 - 0,023 |
| lactate de sodium | 1,35 - 1,65 |
| acétate de sodium | 0,234 - 0,286 |
| L-malate | 0,120 - 0,145 |
| Eau pour préparations injectables | reste jusqu'à 100% |

6. Solution hyperosmolaire combinée pour perfusion selon l'une quelconque des revendications 1 à 4, **caractérisée en ce qu'**elle comprend du xylitol, du chlorure de sodium, du chlorure de potassium, du chlorure de calcium, du chlorure de magnésium, du lactate de sodium, de l'acétate de sodium, du L-malate et de l'eau pour préparations injectables, au rapport de composants suivant en poids sec, % en poids :
| | |
|---|---|
| xylitol | 4 - 6 |
| chlorure de sodium | 0,54 - 0,66 |
| chlorure de potassium | 0,027 - 0,033 |
| chlorure de calcium | 0,008 - 0,012 |
| chlorure de magnésium | 0,017 - 0,023 |
| lactate de sodium | 1,35 - 1,65 |
| acétate de sodium | 0,234 - 0,286 |
| L-malate | 0,120 - 0,145 |
| Eau pour préparations injectables | reste jusqu'à 100% |
